# EUROPÄISCHE PATENTSCHRIFT

(11) **EP 2 023 862 B1**
(45) Veröffentlichungstag und Bekanntmachung des Hinweises auf die Patenterteilung: **02.09.2015**
(21) Anmeldenummer: 07724590.0
(22) Anmeldetag: 25.04.2007
(51) Int. Cl.: A61B 17/86, A61F 2/30, A61F 2/40

(54) **SCHULTERPROTHESE MIT VORSPRUNG AN DER GRUNDPLATTE**
SHOULDER PROSTHESIS COMPRISING A PROTRUSION ON THE BASE PLATE
PROTHÈSE D'ÉPAULE DOTÉE D'UNE PIÈCE EN SAILLIE SUR LA PLAQUE DE BASE

(30) Priorität: 22.05.2006 DE 102006023957; 05.09.2006 DE 102006041550
(43) Veröffentlichungstag der Anmeldung: 18.02.2009
(73) Patentinhaber: Mathys AG Bettlach, 2544 Bettlach (CH)
(72) Erfinder: DALLMANN, Frank, 04626 Schmölln (DE)
(74) Vertreter: Thun, Clemens
(86) Internationale Anmeldenummer: PCT/EP2007/003660
(87) Internationale Veröffentlichungsnummer: WO 2007/134690

(56) Entgegenhaltungen:
- EP-A- 1 520 561
- EP-A- 1 607 070
- EP-A1- 1 064 890
- WO-A-01/47442
- FR-A- 2 103 145
- FR-A1- 2 704 747
- US-A- 4 206 517
- US-B1- 6 790 234

## Beschreibung

Die Erfindung bezieht sich auf eine Schulterprothese mit einem Gelenkkopf und einer mit diesem zusammenwirkenden Gelenkpfanne.

Eine Schulterprothese der erfindungsgemäßen Art ist üblicherweise derart aufgebaut, dass eine mit einem Gelenkkopf zusammenwirkende Gelenkpfanne an einem Oberarmknochen (Humerus) und der Gelenkkopf in einer anatomischen Glenoidpfanne (Cavitas Glenoidalis) eines Schulterblatts (Scapula) befestigt sind. Im Gegensatz zum anatomischen Schultergelenk, bei dem der Humeruskopf des Oberarmknochens mit der Glenoidpfanne (Cavitas Glenoidalis) des Schulterblatts ein Kugelgelenk bildet, stellt diese Schulterprothese ein inverses Schultergelenk dar. Beim Ab- und Anwinkeln des Oberarms an den Oberkörper (Abduktion/Adduktion) dreht sich die am Humerus fixierte Gelenkpfanne um den Gelenkkopf. Ist der Oberarm vergleichsweise stark adduziert, steht ein Teilstück der Gelenkpfanne an einem Gelenkkopfrand derart über, dass ein Gelenkpfannenrand des Teilstücks an einem Unterrand der Cavitas Glenoidalis des Schulterblatts anstößt (Inferior Notching).

Um das Anstoßen der Gelenkpfanne bei vergleichsweise starkem Anwinkeln des Oberarms zu vermeiden, ist es bereits bekannt, den Gelenkkopf in Richtung einer unteren Ecke (Angulus Inferior) des Schulterblatts versetzt an der Cavitas Glenoidalis zu montieren. Aus der EP 1 607 070 A1 ist eine derartige Schulterprothese mit versetztem Gelenkkopf bekannt. Hierbei ist ein zur Verankerung am Schulterblatt vorgesehener Vorsprung zentrisch zur Mittelachse einer Grundplatte schulterblattseitig an dieser angeordnet. Des Weiteren weist der Gelenkkopf eine exzentrisch zu dessen Mittelachse ausgerichtete Aufnahme für die Grundplatte auf. Die EP 1 520 561 A1 offenbart die Merkmale des Oberbegriffs des Anspruchs 1.

Der Erfindung liegt die Aufgabe zu Grunde, eine Schulterprothese mit besonders gleichmäßiger Kräfteverteilung zwischen den Formteilen der Schulterprothese und an Verankerungsschnittstellen der Schulterprothese mit dem anatomischen Schulterblatt zur Erhöhung von Materiallebensdauer und Verankerungssicherheit der Schulterprothese anzugeben. Das sollte auch und insbesondere möglich sein bei einer nach inferior versetzten Positionierung der Glenosphäre zur Reduzierung des Inferior Notching unter Nutzung des besonders stark ausgebildeten Knochenbestandes der zentralen anatomischen Glenoidpfanne.

Die genannte Aufgabe wird erfindungsgemäß gelöst durch die Merkmale des Anspruchs 1. Vorteilhafte Weiterbildungen sind Gegenstand der hierauf rückbezogen Unteransprüche.

So umfasst die Schulterprothese gemäß der vorliegenden Erfindung eine Gelenkpfanne sowie einen mit dieser zusammenwirkenden Gelenkkopf, welcher mit einer scapulaseitigen (schulterblattseitigen) Grundplatte nach Art einer Klemmverbindung zusammenwirkt. Der Gelenkkopf ist mit einer zentrisch zu dessen Mittelachse an diesem ausgebildeten Aufnahme auf die Grundplatte aufgesteckt, welche mit einem an der Grundplatte angeordneten Vorsprung sowie mit Verankerungselementen, welche in an der Grundplatte vorgesehenen Hülsen aufnehmbar sind, an der Cavitas Glenoidalis (anatomischen Gelenkpfanne) der Scapula verankerbar ist. Erfindungsgemäß ist vorgesehen, dass die Grundplatte eine zur Anlage an der Cavitas Glenoidalis der Scapula vorgesehene kalottenförmige Bodenfläche aufweist, wobei das Zentrum der Kalottenform nach superior versetzt exzentrisch zur Mittelachse der Grundplatte angeordnet ist. Vorzugsweise ist hierbei der Vorsprung entlang der Mittelachse der Kalottenform der Bodenfläche angeordnet

Gemäß einer nicht erfindungsgemäßen Schulterprothese umfasst diese eine Gelenkpfanne und einen Gelenkkopf, welcher mit der Gelenkpfanne nach Art eines Kugelgelenks zusammenwirkt, sowie eine Grundplatte, welche scapulaseitig (schulterblattseitig) an der Cavitas Glenoidalis (anatomische Gelenkpfanne) der Scapula (Schulterblatt) mit einem Vorsprung und Verankerungselementen befestigbar und an einer der Gelenkpfanne zugewandten Seite mit dem Gelenkkopf nach Art einer Klemmverbindung verbunden ist, wobei am Gelenkkopf zentrisch zu dessen Mittelachse eine dem Ausmaß der Grundplatte entsprechende Aufnahme ausgebildet ist, um den Gelenkkopf zur Ausbildung der Klemmverbindung auf die Grundplatte aufzustecken. An der Grundplatte sind Hülsen zur Aufnahme der Verankerungselemente und scapulaseitig der Vorsprung exzentrisch zur Mittelachse der Grundplatte und im Bereich des Vorsprungs eine der Hülsen zur Befestigung der Grundplatte an der Cavitas Glenoidalis ausgebildet.

Die mit der Erfindung erzielten Vorteile bestehen zunächst darin, dass mittels der zentrisch zur Mittelachse des Gelenkkopfs an diesem ausgebildeten Aufnahme zur Aufnahme der Grundplatte eine von dem Gelenkkopf auf die Gelenkpfanne ausgeübte Kraft besonders gleichmäßig auf die Grundplatte und die Scapula übertragen wird sowie eine exzentrische Belastung der Klemmverbindung zwischen der Grundplatte und dem Gelenkkopf sowie Materialverschleiß, insbesondere am Kugelgelenk sowie an der Verankerungsschnittstelle zwischen der Grundplatte und der Scapula vermieden wird. Ferner sorgen die Exzentrität der Bodenfläche der Grundplatte bzw. der exzentrisch zur Mittelachse der Grundplatte an dieser angeordnete Vorsprung und eines der Verankerungselemente, welches in einer der Hülsen aufgenommen ist, welche im Bereich des Vorsprungs an der Grundplatte ausgebildet ist, für eine besonders sichere und zuverlässige Verankerung der an der Cavitas Glenoidalis der Scapula versetzt angeordneten Grundplatte. Das gemäß dem zweiten Erfindungsaspekt im Bereich des Vorsprungs vorgesehene Verankerungselement erhöht zudem die Verankerungssicherheit der Grundplatte an der Scapula und mindert außerdem die Belastung des Vorsprungs durch auf den Gelenkkopf und die Grundplatte einwirkende Kräfte. Darüber hinaus werden ein Materialbruch zwischen der Grundplatte und dem Vorsprung und damit ein Ausbrechen der Grundplatte aus der Scapula dadurch verhindert, dass die Verankerungselemente auf den Vorsprung einwirkende Kräfte, welche von der Gelenkpfanne hervorgerufen werden, aufnehmen.

Um ein Anstoßen der Gelenkpfanne bei vergleichsweise starkem Anwinkeln des Oberarms an den Oberkörper wirkungsvoll zu vermeiden, ist die Grundplatte zur versetzten Befestigung vorzugsweise an der Cavitas Glenoidalis in Richtung auf den Angulus Inferior des Schulterblatts ausgestaltet. Dabei ist das Zentrum der Kalottenform der Bodenfläche in eine gegenüber einem Angulus Inferior der Scapula entgegengesetzte Richtung verschoben bzw. der Vorsprung scapulaseitig am oberen, dem Angulus Inferior abgewandten Teilstück der Grundplatte an dieser angeordnet. Die Verankerung des Vorsprungs an der Scapula wird mittels eines Verankerungselements verstärkt, welches in eine im Bereich des Vorsprungs an der Grundplatte ausgebildete Hülse aufnehmbar ist.

Um die Klemmverbindung zwischen dem Gelenkkopf und der Grundplatte bei vergleichsweise starker Belastung des Gelenkkopfs durch die Gelenkpfanne zuverlässig aufrechtzuerhalten, sind eine Mantelfläche der Grundplatte und eine mit dieser die Klemmverbindung bildende Aufnahmeseitenfläche vorzugsweise kreisförmig ausgebildet, so dass eine Gelenkkopfwand zwischen der Aufnahmeseitenfläche und einer Außenfläche des Gelenkkopfs, woran die Gelenkpfanne angreift, eine gleichbleibende Wandstärke aufweist.

Um einem Verdrehen des mit der Grundplatte über die Klemmverbindung zusammenwirkenden Gelenkkopfes auf der Grundplatte durch auf den Gelenkkopf radial einwirkende Kräfte vorzubeugen, weist der Gelenkkopf an einem Teilstück vorzugsweise randseitig eine Nut auf, welche mit einer an der Grundplatte vorgesehenen Feder zusammenwirkt.

Zur besonders sicheren Verankerung der Grundplatte ist die Feder zweckmäßigerweise bevorzugt an dem dem Angulus Inferior abgewandten Teilstück der Grundplatte an dieser angeordnet, wobei im Bereich der Feder eine der Hülsen zur Aufnahme eines der Verankerungselemente an der Grundplatte ausgebildet ist.

Gemäß vorteilhafter Ausgestaltung ist die Aufnahme am Gelenkkopf zur Aufnahme der Grundplatte derart ausgebildet, dass die Grundplatte vollständig darin verschwindet und eine Randfläche des Gelenkkopfs und die Bodenfläche der Grundplatte im wesentlichen bündig ineinander übergehen. Damit ist die scapulaseitige Aufnahme des Gelenkkopfs von der Umgebung abgeschlossen und vor Beschädigung durch Fremdeinwirkung geschützt.

Da die Grundplatte in einer Vormontage zunächst mit der Bodenfläche der Grundplatte an der Cavitas Glenoidalis aufgesetzt und mit dem Vorsprung in der Scapula verankert wird, bevor die Verankerung durch die Verankerungselemente erfolgt, ist es zweckmäßig, den Vorsprung in Längsrichtung rechtwinklig zur Bodenfläche der Grundplatte an dieser anzuordnen.

Zur sicheren Verankerung der Grundplatte ist der Vorsprung bevorzugt nach Art eines Dübels ausgestaltet. Der Vorsprung weist zweckmäßigerweise Rippen auf, welche mit einer Bohrwand einer für den Vorsprung in der Scapula vorgesehenen Bohrung Klemmverbindungen bilden.

Zur Verankerung der Grundplatte sind bevorzugt zusätzlich zum Vorsprung die Verankerungselemente vorgesehen, welche in die Hülsen der Grundplatte einsteckbar sind. Die Hülsen stellen an der Grundplatte ausgebildete Durchführungen von einer der Scapula abwandten Seite zu einer der Scapula zugewandten Seite der Grundplatte dar und weisen an der der Scapula abgewandten Seite zweckmäßigerweise Versenkungen auf, worin die Verankerungselemente mit an diesen vorgesehenen Köpfen lagerbar sind.

Gemäß vorteilhafter Ausgestaltung sind die Hülsen um den Vorsprung liegend an der Grundplatte ausgebildet. Die in den Hülsen aufnehmbaren Verankerungselemente verstärken die Verankerung der Grundplatte und nehmen auf den Vorsprung einwirkende Kräfte auf, um insbesondere die Verankerungssicherheit zu erhöhen sowie einem Materialbruch zwischen dem Vorsprung und der Grundplatte vorzubeugen.

In zweckmäßiger Weiterbildung zur Aufnahme von auf die Grundplatte einwirkenden Kräften, welche durch die Gelenkpfanne hervorgerufen sind, wird die Verankerung der Grundplatte mittels der Verankerungselemente dadurch verstärkt, dass zumindest einige der Verankerungselemente ähnlich den Fingern einer weit geöffneten Hand voneinander weggespreizt in der Scapula fixiert werden. Dementsprechend sind die diese Verankerungselemente führenden Hülsen an der Grundplatte ausgebildet.

Gemäß vorteilhafter Ausgestaltung sind die Verankerungselemente als Verankerungsschrauben ausgeführt.

Um ein Schlackern der Grundplatte auf der Cavitas Glenoidalis zu vermeiden und die Grundplatte besonders sicher und zuverlässig an der Scapula zu fixieren, ist es zweckmäßig, an den Hülsen, die zur Führung der gespreizten Verankerungselemente vorgesehen sind, Schraubgewinde auszubilden, welche mit den Schraubgewinden der als Verankerungsschrauben ausgeführten Verankerungselemente zur Ausbildung von winkelstabilen Schraubverbindungen zusammenwirken. Die weiteren Hülsen sind hingegen vorzugsweise derart ausgeführt, dass für die im wesentlichen parallele Ausrichtung der Verankerungselemente zum Vorsprung ein geringfügiger Spielraum besteht.

In zweckmäßiger Weiterbildung umfasst die Grundplatte gelenkkopfseitig eine Bohrung, welche scapulaseitig bis in den Vorsprung durch die Grundplatte hindurch führt und gelenkkopfseitig eine Verlängerung einer im Gelenkkopf ausgebildeten Durchführung darstellt. Die Durchführung und die Bohrung dienen beispielsweise zur Zentrierung des Gelenkkopfs beim Aufstecken auf die Grundplatte mittels eines darin eingesetzten Zentrierstiftes oder zur zusätzlichen Fixierung des Gelenkkopfs an der Grundplatte beispielsweise mittels eines mit der Durchführung und der Bohrung zusammenwirkenden Klemmwerkstücks.

Nachfolgend wird ein Ausführungsbeispiel der Erfindung anhand einer Zeichnung näher erläutert. Darin zeigen
- Fig. 1: eine Draufsicht einer Schulterprothese mit einer Gelenkpfanne und einem Gelenkkopf sowie einer mit diesem verbundenen Grundplatte mit Verankerungselementen,
- Fig. 2: eine Explosionsdarstellung der Schulterprothese gemäß Fig. 1,
- Fig. 3: eine Draufsicht der Grundplatte gemäß Fig. 1,
- Fig. 4: einen ersten Längsschnitt durch den Gelenkkopf und die mit diesem zusammenwirkende Grundplatte gemäß Fig. 1,
- Fig. 5: einen zweiten Längsschnitt durch den Gelenkkopf und die Grundplatte gemäß Fig. 1,
- Fig. 6: eine Draufsicht einer anatomischen Scapula (Schulterblatt),
- Fig. 7: eine Draufsicht der an der Scapula gemäß Fig. 6 montierten Schulterprothese gemäß Fig. 1 und
- Fig. 8: einen Längsschnitt durch die an der Scapula montierte Schulterprothese gemäß Fig. 7.

Einander entsprechende Teile in allen Figuren mit den gleichen Bezugszeichen versehen.

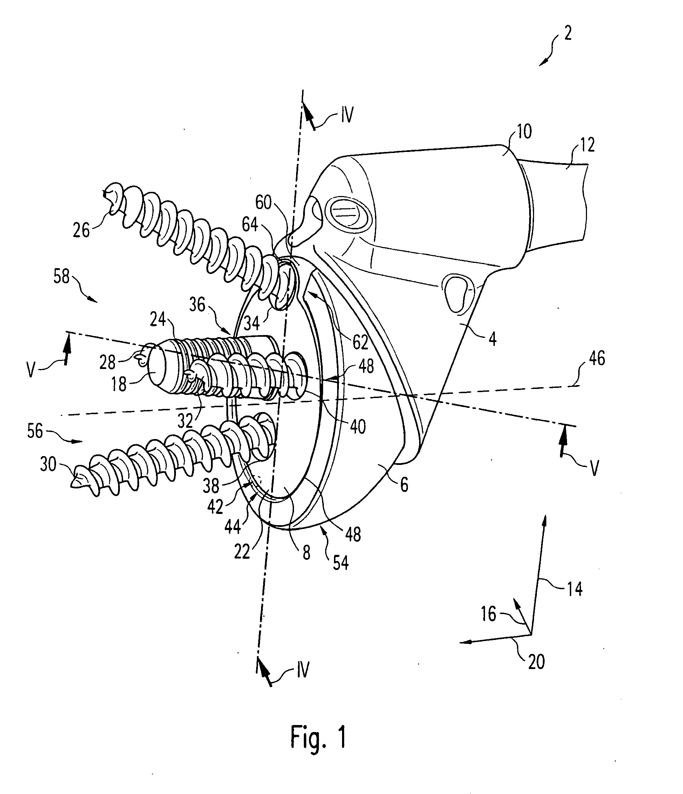

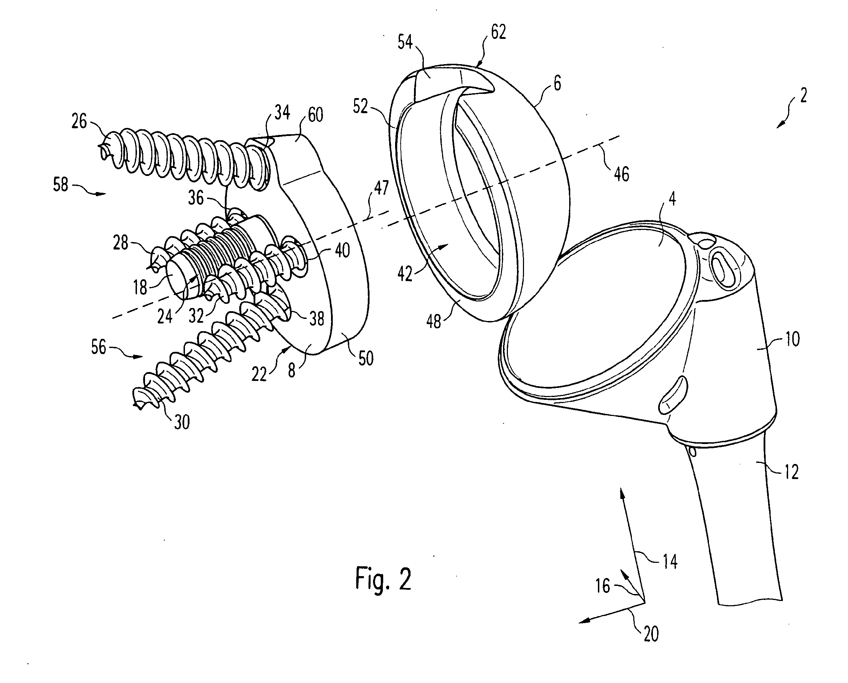

Figuren 1 und 2 zeigen eine Draufsicht bzw. eine Explosionsdarstellung einer Schulterprothese 2 mit einer Gelenkpfanne 4, welche mit einem Gelenkkopf 6 nach Art eines Kugelgelenks zusammenwirkt, und einer Grundplatte 8, worauf der Gelenkkopf 6 aufgesteckt ist. Die Gelenkpfanne 4 ist über ein Kopplungsstück 10 mit einem Stab 12 verbunden, welcher über einen in Figuren 1 und 2 nicht gezeigten Humerusschaft im Oberarm (Humerus) versenkt ist. Die Gelenkpfanne 4 ist in und entgegen der mit den Richtungspfeilen 14 und 16 gekennzeichneten Richtungen auf dem Gelenkkopf 6 schwenkbar vorgesehen. In Fig. 1 ist die Gelenkpfanne 4 in einer Position gezeigt, wobei der in Fig. 1 nicht dargestellte Oberarm in Schulter- oder Kopfhöhe vom Körper weggestreckt ist.

Zur Verankerung der Schulterprothese 2 am in Figuren 1 und 2 nicht gezeigten Schulterblatt (Scapula) ist ein Vorsprung 18 in mit einem Richtungspfeil 20 gekennzeichneter Verankerungsrichtung, rechtwinklig zur einer Bodenfläche 22 der Grundplatte 8 an dieser angeordnet. Um die Grundplatte 8 sicher zu verankern, ist der Vorsprung 18 nach Art eines Dübels ausgebildet und weist Rippen 24 auf. Zusätzlich zum Vorsprung 18 sind Verankerungselemente 26,28,30,32 vorgesehen, welche in an der Grundplatte 8 ausgebildeten Hülsen 34,36,38,40 aufgenommen sind. Die Verankerungselemente 26,28,30,32 sind im gezeigten Ausführungsbeispiel als Verankerungsschrauben ausgeführt. Dabei richten die die Verankerungselemente 26,28,30,32 führenden Hülsen 34,36,38,40 die Verankerungselemente 26,28,30,32 derart aus, dass die obere und untere Schraube 26 und 30 ähnlich den Fingern einer weit geöffneten Hand voneinander weggespreizt sind. Beide winkelstabil geführten Schrauben 26, 30 bewirken ein Verspreizen der Grundplatte 8 mit dem Knochenmaterial des Schulterblatts, wodurch eine nochmals verbesserte Befestigung der Prothese erzielt wird. Die beiden weiteren Schrauben 28 und 32 werden hingegen primär dazu verwendet, die Grundplatte 8 anfänglich an dem Schulterblatt festzuschrauben, und sind hierzu im wesentlichen parallel zu dem Vorsprung 18 ausgerichtet. Im Gegensatz zu den Schrauben 26 und 30 besteht ein gewisser Spielraum für die Ausrichtung dieser weiteren Schrauben 28, 32, wodurch das anfängliche Befestigen der Grundplatte 8 vereinfacht wird.

Der Gelenkkopf 6 umfasst zur Aufnahme der Grundplatte 8 eine Aufnahme 42, deren Ausmaß zur Ausbildung einer Klemmverbindung 44 zwischen dem Gelenkkopf 6 und der Grundplatte 8 ausgelegt ist. Die Aufnahme 42 ist zentrisch zur Mittelachse 46 des Gelenkkopfs 6 an diesem ausgebildet. Wie später noch ausführlicher erläutert wird, weist die Bodenfläche 22 der Grundplatte 8 eine Kalottenform auf, deren Zentrum gegenüber der Mittelachse 47 der Grundplatte versetzt ist. Der Vorsprung 18 ist hierbei vorzugsweise im Zentrum dieser Kalottenform angeordnet und parallel zur Mittelachse 47 der Grundplatte ausgerichtet. Zudem ist die Aufnahme 42 derart dimensioniert, dass die Grundplatte 8 beim Aufstecken des Gelenkkopfs 6 auf die Grundplatte 8 vollständig in der Aufnahme 42 verschwindet.

Zur Ausbildung der Klemmverbindung 44 zwischen dem Gelenkkopf 6 und der Grundplatte 8 wirkt eine Mantelfläche 50 der Grundplatte 8 mit einer Aufnahmeseitenfläche 52 zusammen. Die Mantelfläche 50 und die Aufnahmeseitenfläche 52 sind dabei kreisförmig derart ausgebildet, dass eine durch die Randfläche 48 begrenzte Gelenkkopfwand 54 im Bereich der Randfläche 48 eine gleichbleibende Wandstärke aufweist. Im diesem Zusammenhang ist anzumerken, dass die Verbindung zwischen Grundplatte 8 und Gelenkkopf 6 auch anderweitig durch form- und/oder kraftschlüssig zusammenwirkende Elemente realisiert werden könnte. Zur Herstellung der beiden Elemente können dabei sämtliche in der Prothetik gängigen Materialien eingesetzt werden, wobei durchaus auch die Möglichkeit einer Paarung von Elementen aus verschiedenen Materialien besteht.

Da die Grundplatte 8 auf der in Figuren 1 und 2 nicht dargestellten Cavitas Glenoidalis entgegen der Pfeilrichtung 14 versetzt fixiert ist, kann die Grundplatte 8 an einem unteren Teilstück 56 nicht vollständig mit der Bodenfläche 22 auf der Cavitas Glenoidalis aufliegen. Aufgrund dessen ist die Hülse 34 in einem oberen Teilstück 58 zur Erhöhung der Verankerungsstabilität der Grundplatte 8 im Bereich des exzentrisch zur Mittelachse 47 an der Grundplatte 8 angeordneten Vorsprungs 18 an der Grundplatte 8 zur Aufnahme des Verankerungselements 26 ausgebildet. Um die Hülse 34 am oberen Teilstück 58 der Grundplatte 8 ausbrechsicher an der Grundplatte 8 auszubilden, ist an der Grundplatte 8 eine Vorwölbung bzw. Feder 60 angeordnet, welche den notwendigen Platz für die Hülse 34 zur Verfügung stellt. Die Feder 60 wirkt mit einer am Gelenkkopf vorgesehenen Nut 62 zusammen. Die dadurch ausgebildete Nut-Feder-Verbindung 64 dient auch einem fehlerfreien Aufstecken des Gelenkkopfs 6 auf die Grundplatte 8 und vermeidet ein Verdrehen des Gelenkkopfs 6 auf der Grundplatte 8 durch auf den Gelenkkopf 6 radial einwirkende Kräfte.

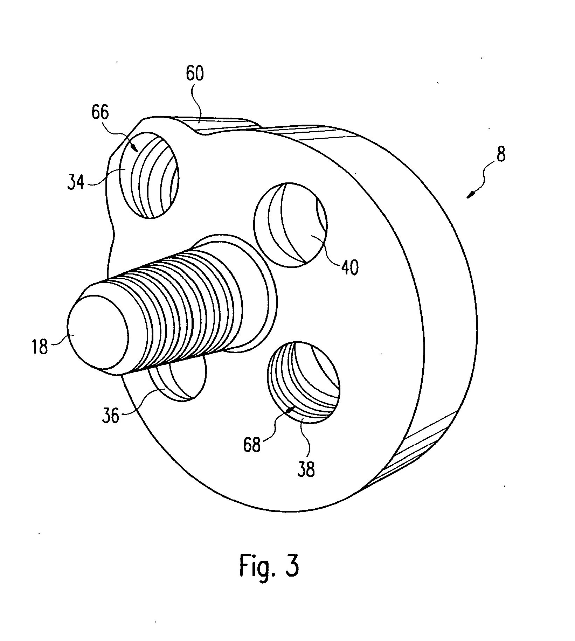

Fig. 3 zeigt eine Draufsicht der Grundplatte 8 gemäß Fig. 1 mit dem Vorsprung 18 und den Hülsen 34,36,38,40 wobei die Hülsen 34 und 38 Schraubgewinde 66,68 aufweisen, welche mit Schraubgewinden der in den Hülsen 34,38 aufnehmbaren, als Verankerungsschrauben ausgeführten Verankerungselemente (in Fig. 3 nicht dargestellt) zur Ausbildung von Schraubverbindungen zusammenwirken. Eine besonders zuverlässige Fixierung der Grundplatte 8 auf der Cavitas Glenoidalis ist mit den Schraubverbindungen zwischen den Hülsen 34,38 und den als Verankerungsschrauben ausgeführten Verankerungselementen sowie zwischen den Verankerungselementen und der Scapula erreichbar. Mittels des in der Hülse 34 aufnehmbaren Verankerungselements ist die Grundplatte 8 im Bereich der Feder 60 zusätzlich fixierbar.

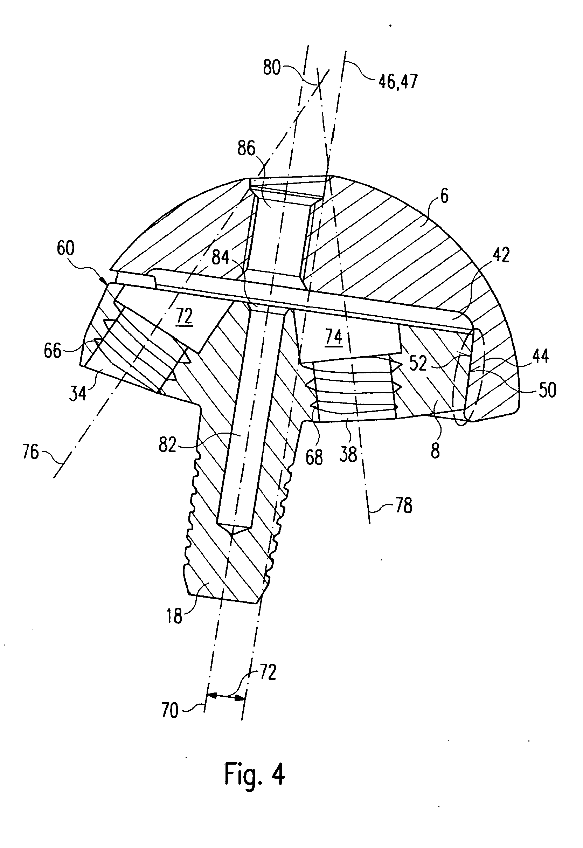

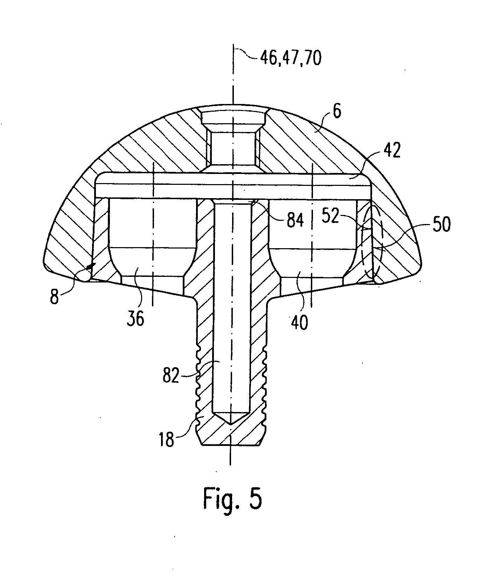

Die Fig. 4 und 5 zeigen zwei Längsschnitte durch den Gelenkkopf 6 und die mit diesem zusammenwirkende Grundplatte 8 gemäß Fig. 1. Der Gelenkkopf 6 umfasst die Aufnahme 42, welche - wie insbesondere der Darstellung von Fig. 4 entnommen werden kann - zentrisch zu dessen Mittelachse 46 an diesem ausgebildet ist. Im Weiteren zeigen die Fig. 4 und 5 die Klemmverbindung 44 zwischen der Aufnahmeseitenfläche 52 des Gelenkkopfs 6 und der Mantelfläche 50 der Grundplatte 8 bei auf die Grundplatte 8 aufgestecktem Gelenkkopf 6.

Der Vorsprung 18 ist entsprechend der Darstellung von Fig. 4 exzentrisch zur Mittelachse 47 der Grundplatte 8 angeordnet, die aufgrund der zentrischen Verbindung zwischen Gelenkkopf 6 und Grundplatte 8 genau auf der Mittelachse 46 des Gelenkkopfs 6 liegt, wobei eine Mittelachse 70 des Vorsprungs 18 zur Mittelachse 47 der Grundplatte 8 parallel verläuft und um einen Abstand 72 von der Mittelachse 47 der Grundplatte 8 beabstandet ist. Bei der Schnittansicht von Fig.5 fallen alle drei Achsen 46, 47 und 70 zusammen. Von besonderer Bedeutung ist hierbei, dass die Bodenfläche 22 der Grundplatte 8, welche im montierten Zustand der Schulterprothese 2 an der Cavitas Glenoidalis des Schulterblatts anliegt, eine kalottenförmige Oberfläche aufweist. Die Mittelachse dieser Kalotte fällt allerdings nicht mit der Mittelachse 47 der Grundplatte 8 zusammen sondern ist stattdessen gegenüber dieser versetzt. Sie bildet genau genommen die Achse 70, entlang der der Vorsprung 18 angeordnet ist. Durch diese besondere Ausgestaltung der Bodenfläche 22 ergeben sich besondere Vorteile hinsichtlich der Montage des Gelenkkopfs 6 an dem Schulterblatt, welche später noch näher erläutert werden.

In den Fig. 4 und 5 sind ferner Längsschnitte der Hülsen 34, 38 bzw. 36 und 40 mit den Schraubgewinden 66, 68 der ersten beiden Hülsen 34, 38 gezeigt, wobei die Hülse 34 im Bereich der Feder 60 an der Grundplatte 8 ausgebildet ist. Zusätzlich zu den Schraubgewinden weisen die Hülsen Versenkungen 72,74 auf, in denen die in den Fig. 4 und 5 nicht gezeigten Verankerungselemente mit an diesen angeordneten Köpfen gelagert sind. Die Hülsen 34, und 38 sind dabei derart an der Grundplatte 8 ausgebildet, dass die in den Hülsen 34, 38 aufnehmbaren Verankerungselemente scapulaseitig wie zwei Finger einer weit geöffneten Hand voneinander wegspreizen. Dazu sind die Hülsen 34,38 hinsichtlich der Mittelachse 47 der Grundplatte 8 in Längsrichtung derart angewinkelt an der Grundplatte 8 ausgebildet, dass Mittelachsen 76,78 der Hülsen 34,38 sich in Verlängerung gelenkkopfseitig schneiden. In Fig. 4 ist ein durch die Mittelachsen 76,78 der Hülsen 34,38 gebildeter Schnittpunkt 80 angedeutet.

Im Gegensatz zu den Hülsen 34 und 38 für die winkelstabil gelagerten Schrauben 26 und 30 sind die beiden weiteren Hülsen 36 und 40 entsprechend der Darstellung in Fig. 5 derart ausgestaltet, dass die zugehörigen Schrauben im wesentlichen parallel zum Vorsprung 18 ausgerichtet sind. Die in diesen Hülsen 36 und 40 geführten Schrauben 28 und 32 dienen dazu, die Grundplatte 8 an dem Schulterblatt zunächst festzuschrauben, bevor mit Hilfe der beiden Schrauben 26 und 32 die Spreizverbindung hervorgerufen wird. Die Hülsen 36 und 40 weisen deshalb keine Gewinde auf und sind ferner in ihrem Kopfbereich kugelabschnittförmig ausgebildet. Werden als Verankerungselemente Schrauben mit einem kugelig ausgebildeten Kopf verwendet, so können diese in einem kleinen Winkelbreich geschwenkt werden. Dieser geringfügige Spielraum ermöglicht ein einfacheres Einbringen der Schrauben in das Schulterblatt.

Zusätzlich zu den Hülsen 34,36,38 und 40 ist in der Grundplatte 8 eine Bohrung 82 vorgesehen, welche gelenkkopfseitig ein offenes Ende 84 aufweist und scapulaseitig bis in den Vorsprung 18 hineinreicht. In Verlängerung der Bohrung 82 ist im Gelenkkopf 6 eine Durchführung 86 ausgebildet. Die Durchführung 86 und die Bohrung 82 dienen beispielsweise zur Aufnahme eines in den Fig. 4 und 5 nicht gezeigten Zentrierstifts zur Zentrierung des Gelenkkopfs 6 beim Aufstecken auf die Grundplatte 8 oder zur Aufnahme eines in den Fig. 4 und 5 nicht gezeigten, mit der Durchführung 86 und der Bohrung 82 zusammenwirkenden Klemmwerkstücks zur zusätzlichen Fixierung des Gelenkkopf 6 an der Grundplatte 8.

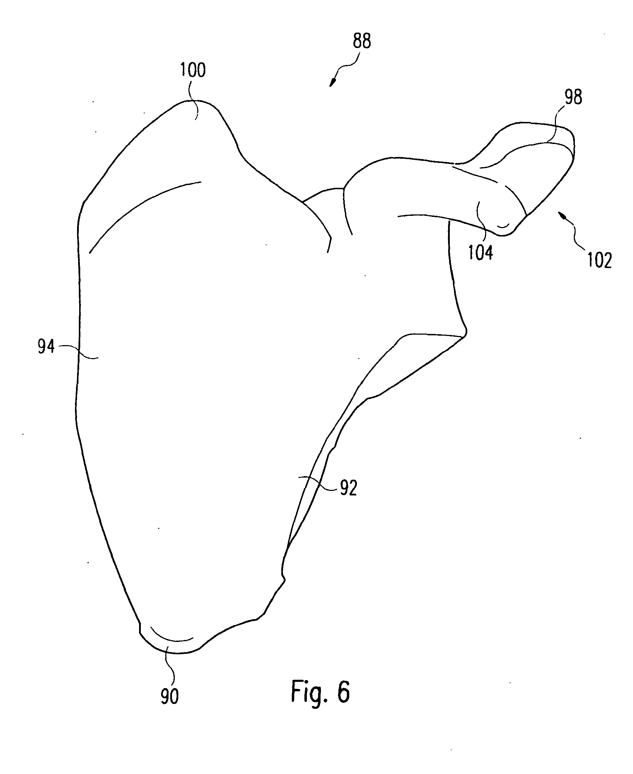

Fig. 6 zeigt eine Draufsicht einer anatomischen Scapula 88 (Schulterblatt), deren Grundform ein Dreieck darstellt. Dabei wird das untere Eck des Dreiecks als Angulus Inferior 90 bezeichnet, woran sich ein rechter Rand "Margo Lateralis 92" und ein linker Rand "Margo Medialis 94" anschließen. Der Margo Lateralis 92 und der Margo Medialis 94 begrenzen den oberen Rand "Margo Superior 96" an einem rechten Eck "Angulus Lateralis 98" bzw. an einem linken Eck "Angulus Superior 100". Am Angulus Lateralis 98 ist eine anatomische Gelenkpfanne "Cavitas Glenoidalis 102" positioniert, welche einen nach vorne und außen gekrümmten Rabenschnabelfortsatz "Processus Coracoideus 104" aufweist.

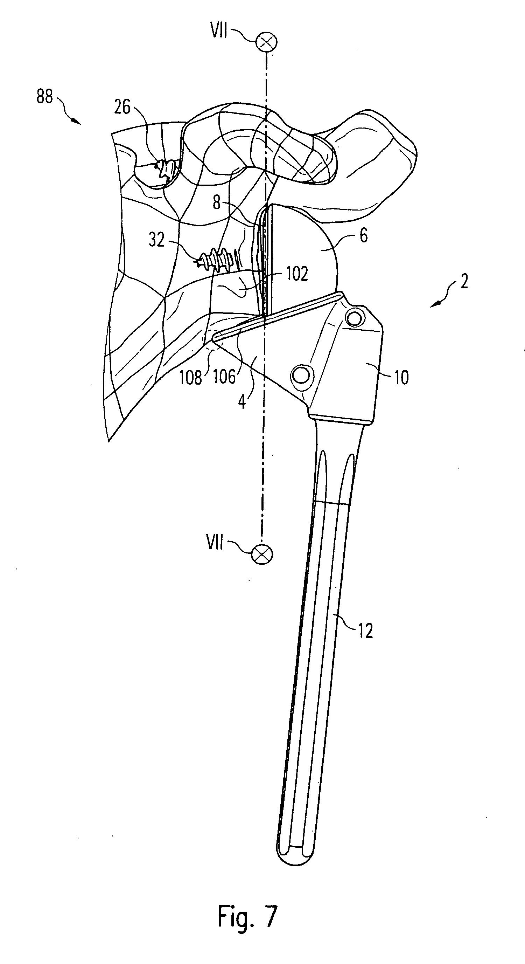

In Fig. 7 ist eine Draufsicht der an der Scapula 88 gemäß Fig. 6 montierten Schulterprothese 2 gemäß Fig. 1 mit Gelenkkopf 6 und Grundplatte 8 gezeigt. Die über das Kopplungsstück 10 am Stab 12 befestigte Gelenkpfanne 4 ist am in Fig. 7 nicht gezeigten Oberarmknochen (Humerus) fixiert. Die Grundplatte 8 ist an der Cavitas Glenoidalis 102 der Scapula 88 mit dem in Fig. 7 nicht gezeigten Vorsprung und Verankerungselementen verankert. In Fig. 7 sind lediglich Enden der als Verankerungsschrauben ausgeführten Verankerungselemente 26 und 32 gezeigt.

In Fig. 7 ist die Gelenkpfanne 4 in der Position des an den Oberkörper angewinkelten Oberarmes gezeigt, wobei mit der Grundplatte 8, welche in Richtung des Angulus Inferior 90 versetzt an der Cavitas Glenoidalis 102 angeordnet ist, ein Anstoßen eines Gelenkpfannenrandes 106 bei vergleichsweise stark angewinkeltem Oberam vermieden wird. In Fig. 7 ist am Unterrand der Cavitas Glenoidalis 102, welcher an den Margo Lateralis 92 der Scapula 88 anschließt, eine Kontaktstelle 108 gezeigt, woran der Gelenkpfannenrand 106 für den Fall anstößt, dass der Oberarm unverhältnismäßig stark angewinkelt wird.

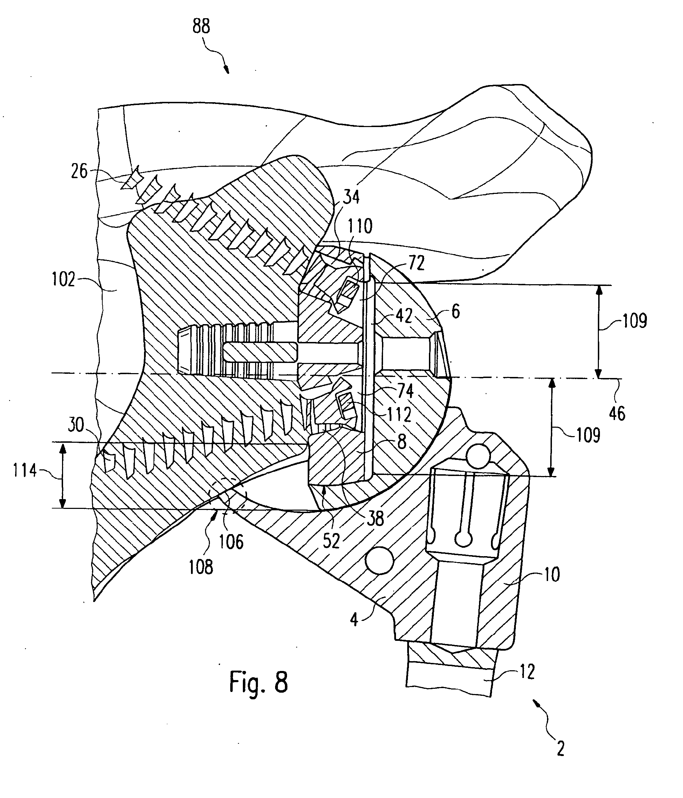

In Fig. 8 ist ein Längsschnitt durch die an der Scapula 88 montierte Schulterprothese 2 gemäß Fig. 7 mit dem Gelenkkopf 6 und der Grundplatte 8 sowie der Gelenkpfanne 4 gezeigt, welche über das Kopplungsstück 10 und den Stab 12 am in Fig. 8 nicht gezeigten Humerus gehalten ist. Der Gelenkkopf 6 ist mit der Aufnahme 42 auf die Grundplatte 8 aufgesteckt. Die Aufnahme 42 ist zentrisch um die Mittelachse 46 des Gelenkkopfs 6 an diesem ausgebildet und seitlich von einer Aufnahmeseitenfläche.52 begrenzt, welche kreisförmig um die Mittelachse 46, in dieser Ausführung mit Radius 109, verläuft.

Die Grundplatte 8 ist mit dem Vorsprung 18 und den Verankerungselementen an der Cavitas Glenoidalis 102 verankert. In Fig. 8 sind lediglich die als Verankerungsschrauben ausgeführten Verankerungselemente 26 und 30 gezeigt, welche gelenkkopfseitig in die Hülsen 34,38 der Grundplatte 8 aufgenommen und mit an den Verankerungselementen 26,30 angeordneten Köpfen 110,112 in den Versenkungen 72,74 der Hülsen 34,38 gelagert sind. Dabei ist die Hülse 34 im Bereich der Feder 62 an der Grundplatte 8 ausgebildet, um die Grundplatte 8 an der dem in Fig. 8 nicht gezeigten Angulus Inferior abgewandten Seite an der Cavitas Glenoidalis 102 der Scapula 88 zusätzlich zu fixieren.

Die Grundplatte 8 ist, wie in Fig. 8 gezeigt, in Richtung des Angulus Inferior 90 um eine Distanz 114 versetzt an der Cavitas Glenoidalis 102 angeordnet, wodurch ein Anstoßen des Gelenkpfannenrands 106 bei zum Oberkörper angewinkeltem Oberarm vermieden wird. Lediglich bei verhältnismäßig starkem Anwinkeln des Oberarms ist ein Anstoßen des Gelenkpfannenrands 106 an der Kontaktstelle 108 der Cavitas Glenoidalis 102 nicht ausgeschlossen.

Diese versetzte Anordnung der Grundplatte 8 wird dabei insbesondere dadurch begünstigt, dass - wie zuvor erläutert - das Zentrum der Kalottenform der Bodenfläche 22 der Grundplatte 8 exzentrisch zu deren Zentrum angeordnet ist. Insbesondere ist dieses Zentrum in entgegengesetzter Richtung gegenüber dem Angulus Inferior 90 der Scapula 88 verschoben. Diese besondere Ausgestaltung hat ferner auch zur Folge, dass die Oberfläche der Cavitas Glenoidalis 102 eine für eine Montage der Schulterprothese 2 vorteilhafte kugelabschnitt-förmige Vertiefung aufweisen kann, da das Zentrum der Auflagefläche der Bodenfläche 22 mit dem Zentrum der Kalottenform zusammenfällt. Die vor der Montage erforderliche Bearbeitung der Cavitas Glenoidalis 102 ist deshalb für einen Operateur im Vergleich zu bislang bekannten Lösung besonders einfach, wobei dennoch ein vollständiges Anliegen der Bodenfläche 22 an der Cavitas Glenoidalis 102 über die gesamte Kontaktfläche hinweg erzielt werden kann. Wesentlich hierbei ist, dass die Form der Grundplatte 8 in optimaler Weise an die gegebenen Knochenverhältnisse angepasst ist, wodurch eine einfache aber auch besonders sichere Befestigung der Prothese an dem Schulterblatt ermöglicht wird. Dies stellt einen entscheidenden Vorteil im Vergleich zu Lösungen aus dem Stand der Technik dar, bei denen der Knochen stark bearbeitet bzw. manipuliert werden muss, um ein Befestigen der Prothese zu ermöglichen.

Da ferner der Vorsprung 18 entlang der Längsachse 70 der Kalottenform der Bodenfläche 22 und damit im Zentrum der Kontaktfläche zwischen Bodenfläche 22 und Cavitas Glenoidalis 102 angeordnet ist, kann eine zentrale und damit optimale Kraftübertragung, welche eine zuverlässige Verankerung des Gelenkkopfs 6 an der Scapula 88 ermöglicht, sichergestellt werden. Insbesondere Scherkräfte werden in diesem Fall besonders gut erfasst, ohne das die Gefahr einer Lockerung besteht. Die erfindungsgemäße Lösung erlaubt deshalb nicht nur eine einfach durchzuführende sondernd auch eine besonders zuverlässige Montage des Gelenkkopfs 6 am Schulterblatt 2.

Die Erfindung ist nicht auf das in der Zeichnung dargestellte Ausführungsbeispiel beschränkt. Alle vorstehend beschriebenen und in der Zeichnung dargestellten Merkmale sind beliebig miteinander kombinierbar.

## Patentansprüche

1. Schulterprothese (2) mit einer Gelenkpfanne (4) und einem mit dieser zusammenwirkenden Gelenkkopf (6), welcher mit einer scapulaseitigen Grundplatte (8) nach Art einer Klemmverbindung (44) zusammenwirkt, wobei der Gelenkkopf (6) mit einer zentrisch zu dessen Mittelachse (46) an diesem ausgebildeten Aufnahme (42) auf die Grundplatte (8) aufgesteckt ist, welche mit einem an der Grundplatte angeordneten Vorsprung (18) sowie mit Verankerungselementen (26, 28, 30, 32), welche in an der Grundplatte (8) vorgesehenen Hülsen aufnehmbar sind, an der Cavitas Glenoidalis (102) der Scapula (88) verankerbar ist, und wobei
die Grundplatte (8) eine zur Anlage an der Cavitas Glenoidalis (102) der Scapula (88) vorgesehene kalottenförmige Bodenfläche (22) aufweist, **dadurch gekennzeichnet, dass** das Zentrum der Kalottenform exzentrisch zur Mittelachse (47) der Grundplatte (8) angeordnet ist.

2. Schulterprothese nach Anspruch 1,
**dadurch gekennzeichnet,**
**dass** der Vorsprung (18) entlang der Mittelachse (70) der Kalottenform der Bodenfläche (22) angeordnet ist.

3. Schulterprothese nach Anspruch 1 oder 2,
**dadurch gekennzeichnet,**
**dass** das Zentrum der Kalottenform der Bodenfläche (22) in eine gegenüber einem Angulus Inferior (90) der Scapula (88) entgegengesetzte Richtung verschoben ist.

4. Schulterprothese nach Anspruch 2 oder 3,
**dadurch gekennzeichnet,**
**dass** eine der Hülsen (34) zur Aufnahme eines Verankerungselements (26) im Bereich des Vorsprungs (18) an der Grundplatte (8) ausgebildet ist.

5. Schulterprothese nach einem der Ansprüche 1 bis 4,
**dadurch gekennzeichnet,**
**dass** eine Aufnahmeseitenfläche (52) am Gelenkkopf (6) und eine mit dieser zusammenwirkende Mantelfläche (50) der Grundplatte (8) zur Ausbildung der Klemmverbindung (44) kreisförmig ausgebildet sind.

6. Schulterprothese nach einem der Ansprüche 1 bis 5,
**dadurch gekennzeichnet,**
**dass** an einer dem Angulus Inferior (90) der Scapula (88) abgewandten Seite am Gelenkkopf (6) eine Nut (62) vorgesehen ist, womit die Grundplatte (8) mit einer an dieser ausgebildeten Feder (60) zusammenwirkt.

7. Schulterprothese nach Anspruch 6,
**gekennzeichnet durch**
eine im Bereich der Feder (60) an der Grundplatte (8) ausgebildete Hülse (34).

8. Schulterprothese nach Anspruch 7,
**dadurch gekennzeichnet,**
**dass** der Vorsprung (18) in einer Längsachse rechtwinklig zur Bodenfläche (22) der Grundplatte (8) an dieser angeordnet ist.

9. Schulterprothese nach einem der Ansprüche 1 bis 8,
**dadurch gekennzeichnet,**
**dass** der Vorsprung (18) nach Art eines Dübels Rippen (24) aufweist.

10. Schulterprothese nach einem der Ansprüche 1 bis 9,
**dadurch gekennzeichnet,**
**dass** die Hülsen (34, 36, 38, 40) an einer der Scapula (88) abgewandten Seite der Grundplatte (8) Versenkungen (70, 72) für an den Verankerungselementen (26, 28, 30, 32) vorgesehene Köpfe (110, 112) aufweisen.

11. Schulterprothese nach einem der Ansprüche 1 bis 10,
**dadurch gekennzeichnet,**
**dass** die Hülsen (34, 36, 38, 40) um den Vorsprung (18) liegend an der Grundplatte (8) ausgebildet sind.

12. Schulterprothese nach einem der Ansprüche 1 bis 11,
**dadurch gekennzeichnet,**
**dass** zumindest einige der Hülsen (34, 36, 38, 40) an der Grundplatte (8) zur Führung der Verankerungselemente (26,28,30,32) derart ausgebildet sind, dass die zugehörigen Verankerungselemente (26, 28, 30, 32) bei endmontierter Schulterprothese (2) nach Art von Fingern einer weit geöffneten Hand voneinander weggespreizt sind.

13. Schulterprothese nach Anspruch 12,
**dadurch gekennzeichnet,**
**dass** die weiteren Hülsen (36, 40) an der Grundplatte (8) zur Führung der Verankerungselemente (26, 28, 30, 32) derart ausgebildet sind, dass die zugehörigen Verankerungselemente (28, 32) mit einem geringfügigen Spielraum im wesentlichen parallel zum Vorsprung (18) ausgerichtet sind.

14. Schulterprothese nach einem der Ansprüche 1 bis 13,
**dadurch gekennzeichnet,**
**dass** die Verankerungselemente (26, 28, 30, 32) als Verankerungsschrauben ausgebildet sind.

15. Schulterprothese nach Anspruch 12 oder 13 und Anspruch 14,
**dadurch gekennzeichnet,**
**dass** an den Hülsen (34, 38) zur Aufnahme der gespreizten Verankerungsschrauben (26, 30) Schraubgewinde (66, 68) zur Ausbildung von Schraubverbindungen zwischen der Grundplatte (8) und den Verankerungsschrauben (26, 30) vorgesehen sind.

16. Schulterprothese nach einem der Ansprüche 1 bis 15,
**dadurch gekennzeichnet,**
**dass** in Verlängerung zu einer am Gelenkkopf (6) vorgesehenen Durchführung (86) an der Grundplatte (8) eine Bohrung (82) im Bereich des Vorsprungs (18) ausgebildet ist.

## Claims

1. A shoulder prosthesis (2) having an articulation socket (4) and a condyle (6) which co-operates therewith and which co-operates with a scapula-side base plate (8) in the manner of a clamping connection (44), the condyle (6), by means of a receptacle (42) formed thereon centrally relative to its centre axis (46), being fitted to the base plate (8) which can be anchored to the Cavitas Glenoidalis (102) of the scapula (88) by means of a protrusion (18) which is arranged on the base plate and anchoring elements (26, 28, 30, 32) which can be received in sleeves which are provided on the base plate (8), and wherein the base plate (8) has a dome-like base face (22) which is provided for abutment against the Cavitas Glenoidalis (102) of the scapula (88),
**characterised in that**
the centre of the dome-like shape is arranged eccentrically relative to the centre axis (47) of the base plate (8).

2. A shoulder prosthesis according to claim 1,
**characterised in that**
the protrusion (18) is arranged along the centre axis (70) of the dome-like shape of the base face (22).

3. A shoulder prosthesis according to claim 1 or 2,
**characterised in that**
the centre of the dome-like shape of the base face (22) is displaced in the opposite direction to an Angulus Inferior (90) of the scapula (88).

4. A shoulder prosthesis according to claim 2 or 3,
**characterised in that**
one of the sleeves (34) for receiving an anchoring element (26) is formed in the region of the protrusion (18) on the base plate (8).

5. A shoulder prosthesis according to any one of claims 1 to 4,
**characterised in that**
a receptacle side face (52) on the condyle (6) and an outer face (50) of the base plate (8) that co-operates therewith are constructed in a circular manner in order to form the clamping connection (44).

6. A shoulder prosthesis according to any one of claims 1 to 5,
**characterised in that**
at a side facing away from the Angulus Inferior (90) of the scapula (88), a groove (62) is provided on the condyle (6), with which the base plate (8) co-operates by way of a tongue (60) which is formed thereon.

7. A shoulder prosthesis according to claim 6,
**characterised by**
a sleeve (34) which is formed in the region of the tongue (60) on the base plate (8).

8. A shoulder prosthesis according to claim 7,
**characterised in that**
the protrusion (18) is arranged on the base plate (8) in a longitudinal axis at right-angles to the base face (22) of the base plate (8).

9. A shoulder prosthesis according to any one of claims 1 to 8,
**characterised in that**
the protrusion (18) has ribs (24) in the manner of a peg.

10. A shoulder prosthesis according to any one of claims 1 to 9,
**characterised in that**
the sleeves (34, 36, 38, 40), at a side of the base plate (8) facing away from the scapula (88), have recesses (70, 72) for heads (110, 112) which are provided on the anchoring elements (26, 28, 30, 32).

11. A shoulder prosthesis according to any one of claims 1 to 10,
**characterised in that**
the sleeves (34, 36, 38, 40) are formed on the base plate (8) around the protrusion (18).

12. A shoulder prosthesis according to any one of claims 1 to 11,
**characterised in that**
at least some of the sleeves (34, 36, 38, 40) on the base plate (8) for guiding the anchoring elements (26, 28, 30, 32) are constructed in such a manner that the associated anchoring elements (26, 28, 30, 32) are spread apart from each other in the manner of fingers of a wide-open hand when the shoulder prosthesis (2) is in the final assembled state.

13. A shoulder prosthesis according to claim 12,
**characterised in that**
the additional sleeves (36, 40) on the base plate (8) for guiding the anchoring elements (26, 28, 30, 32) are constructed in such a manner that the associated anchoring elements (28, 32) are orientated with a slight clearance substantially parallel to the protrusion (18).

14. A shoulder prosthesis according to any one of claims 1 to 13,
**characterised in that**
the anchoring elements (26, 28, 30, 32) are constructed as anchoring screws.

15. A shoulder prosthesis according to claim 12 or 13 and claim 14,
**characterised in that**
on the sleeves (34, 38) for receiving the splayed anchoring screws (26, 30), screw threads (66, 68) are provided in order to form screw connections between the base plate (8) and the anchoring screws (26, 30).

16. A shoulder prosthesis according to any one of claims 1 to 15,
**characterised in that**
in continuation of a passage (86) which is provided on the condyle (6) a bore (82) is formed on the base plate (8) in the region of the protrusion (18).

## Revendications

1. Prothèse de l'épaule (2) comprenant un cotyle (4) et une tête d'articulation (6) en coopération avec ce dernier, qui coopère, à la manière d'une liaison par serrage (44), avec une plaque de base (8) côté omoplate, sachant que la tête d'articulation (6) est emboîtée sur la plaque de base (8) par un logement (42) réalisé au niveau de l'axe médian (46) de la tête d'articulation, de manière centrée par rapport à ce dernier, lequel logement peut être ancré, avec une partie faisant saillie (18) disposée au niveau de la plaque de base ainsi qu'avec des éléments d'ancrage (26, 28, 30, 32), qui peuvent être logés dans des douilles prévues au niveau de la plaque de base (8), au niveau de la cavité glénoïde (102) de l'omoplate (88), et
sachant que la plaque de base (8) présente une surface de fond (22) en forme de calotte prévue pour venir en appui au niveau de la cavité glénoïde (102) de l'omoplate (88),
**caractérisée en ce que** le centre de la forme de calotte est disposé de manière excentrée par rapport à l'axe médian (47) de la plaque de base (8).

2. Prothèse de l'épaule selon la revendication 1,
**caractérisée en ce**
**que** la partie faisant saillie (18) est disposée le long de l'axe médian (70) de la forme de calotte de la surface de fond (22).

3. Prothèse de l'épaule selon la revendication 1 ou 2,
**caractérisée en ce**
**que** le centre de la forme de calotte de la surface de fond (22) est déplacé par coulissement dans une direction opposée par rapport à l'angle inférieur (90) de l'omoplate (88).

4. Prothèse d'épaule selon la revendication 2 ou 3,
**caractérisée en ce**
**qu'**une des douilles (34) servant à recevoir un élément d'ancrage (26) est réalisée dans la zone de la partie faisant saillie (18) au niveau de la plaque de base (8).

5. Prothèse d'épaule selon l'une quelconque des revendications 1 à 4,
**caractérisée en ce**
**qu'**une surface latérale de logement (52) au niveau de la tête d'articulation (6) et une surface d'enveloppe (50), coopérant avec cette dernière, de la plaque de base (8) sont réalisées de manière à présenter une forme circulaire afin de réaliser la liaison par serrage (44).

6. Prothèse de l'épaule selon l'une quelconque des revendications 1 à 5,
**caractérisée en ce**
**qu'**une rainure (62) est prévue au niveau d'un côté opposé à l'angle inférieur (90) de l'omoplate (88), au niveau de la tête d'articulation (6), ce qui permet à la plaque de base (8) de coopérer avec un ressort (60) réalisé au niveau de ladite rainure.

7. Prothèse de l'épaule selon la revendication 6,
**caractérisée par**
une douille (34) réalisée dans la zone du ressort (60) au niveau de la plaque de base (8).

8. Prothèse de l'épaule selon la revendication 7,
**caractérisée en ce**
**que** la partie faisant saillie (18) est disposée dans un axe longitudinal, à angle droit par rapport à la surface de fond (22) de la plaque de base (8), au niveau de cette dernière.

9. Prothèse d'épaule selon l'une quelconque des revendications 1 à 8,
**caractérisée en ce**
**que** la partie faisant saillie (18) présente à la manière d'une cheville des nervures (24).

10. Prothèse d'épaule selon l'une quelconque des revendications 1 à 9,
**caractérisée en ce**
**que** les douilles (34, 36, 38, 40) présentent au niveau d'un côté, opposé à l'omoplate (88), de la plaque de base (8), des noyures (70, 72) destinées à des têtes (110, 112) prévues au niveau des éléments d'ancrage (26, 28, 30, 32).

11. Prothèse de l'épaule selon l'une quelconque des revendications 1 à 10,
**caractérisée en ce**
**que** les douilles (34, 36, 38, 40) sont réalisées au niveau de la plaque de base (8) autour de la partie faisant saillie (18).

12. Prothèse de l'épaule selon l'une quelconque des revendications 1 à 11,
**caractérisée en ce**
**qu'**au moins quelques-unes des douilles (34, 36, 38, 40) sont réalisées au niveau de la plaque de base (8) afin de guider des éléments d'ancrage (26, 28, 30, 32) de telle manière que les éléments d'ancrage (26, 28, 30, 32) associés sont écartés les uns des autres dans le cas d'une prothèse d'épaule (2) montée à la manière de doigts d'une main grande ouverte.

13. Prothèse d'épaule selon la revendication 12,
**caractérisée en ce**
**que** les autres douilles (36, 40) sont réalisées au niveau de la plaque de base (8) afin de guider les éléments d'ancrage (26, 28, 30, 32) de telle manière que les éléments d'ancrage (28, 32) associés sont orientés, avec une légère marge de manoeuvre, essentiellement de manière parallèle par rapport à la partie faisant saillie (18).

14. Prothèse de l'épaule selon l'une quelconque des revendications 1 à 13,
**caractérisée en ce**
**que** les éléments d'ancrage (26, 28, 30, 32) sont réalisés sous la forme de vis d'ancrage.

15. Prothèse d'épaule selon la revendication 12 ou 13 et selon la revendication 14,
**caractérisée en ce**
**que** sont prévus, au niveau des douilles (34, 38) servant à recevoir les vis d'ancrage (26, 30) écartées, des filetages de vissage (66, 68) servant à réaliser des liaisons par vissage entre la plaque de base (8) et les vis d'ancrage (26, 30).

16. Prothèse d'épaule selon l'une quelconque des revendications 1 à 15,
**caractérisée en ce**
**qu'**un alésage (82) est réalisé dans la zone de la partie faisant saillie (18) au niveau de la plaque de base (8), dans le prolongement d'un passage (86) prévu au niveau de la tête d'articulation (6).
